# EUROPÄISCHE PATENTSCHRIFT

(11) **EP 0 447 360 B1**
(45) Veröffentlichungstag und Bekanntmachung des Hinweises auf die Patenterteilung: **08.02.1995**
(21) Anmeldenummer: 91810151.0
(22) Anmeldetag: 06.03.1991
(51) Int. Cl.: C08G 59/32, C08G 59/40, C08G 59/12

(54) **Glycidylester von Tricarbonsäureaddukten**
Glycidylester of tricarboxylic acid adducts
Ester glycidylique de produits d'addition à base d'acides tricarboxyliques

(30) Priorität: 15.03.1990 US 494194
(43) Veröffentlichungstag der Anmeldung: 18.09.1991
(73) Patentinhaber: CIBA-GEIGY AG, 4002 Basel (CH)
(72) Erfinder: Brytus, Vincent, Mahopac, N.Y. 10541 (US)

(56) Entgegenhaltungen:
- DE-A- 2 133 039
- PATENT ABSTRACTS OF JAPAN vol. 012, no. 139 (C-491)27. April 1988 & JP-A-62256815

## Beschreibung

Die vorliegende Erfindung betrifft Glycidylester bestimmter Tricarbonsäureaddukte sowie Zusammensetzungen enthaltend derartige Ester.

Die Bildung von Addukten aus 2,2-Dimethylolalkansäuren mit Anhydriden führt zu Zwischenprodukten, die zur Herstellung der erfindungsgemässen Glycidylester benötigt werden können. So führt z.B. die Umsetzung von einem Mol 2,2-Dimethylolpropionsäure (DMPA) mit Phthalsäureanhydrid zu einem Tricarbonsäureaddukt folgender Struktur: 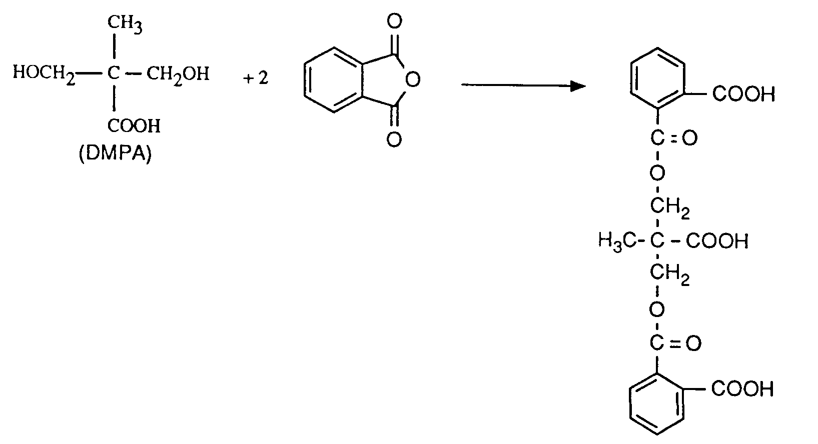
Im US-Patent Nr. 3,404,018 sind Reaktionsprodukte von Hydroxycarbonsäuren mit flüssigen Epoxidharzen (handelsüblichen Bisphenol-A-diglycidylethern) beschrieben. Als Hydroxycarbonsäuren werden 2,2-Dimethylolpropionsäure sowie Addukte von Polyolen mit Dicarbonsäureanhydriden verwendet, wobei als Polyole Ethylenglykol, Propylenglykol, Butandiol, Polyethylenglykole und Glycerin, und als Anhydride Maleinsäure-, Bernsteinsäure-, Dodecenyl-bernsteinsäure-, Phthalsäure-, Tetrahydrophthalsäure-, Hexahydrophthalsäure-, Dichlormaleinsäure- und Hexachlor-endomethylen-tetrahydrophthalsäure-anhydrid eingesetzt werden.

Die Polyole werden mit den Anhydriden in einem 1:1-Mol-Verhältnis umgesetzt, wobei sich der Anhydridring öffnet, was zur Bildung einer Estergruppe zwischen einer Carboxylgruppe des Anhydrids und einer Hydroxylgruppe des Polyols führt, und die restlichen Carboxyl- und Hydroxylgruppen bleiben unverestert.

Die Reaktion der erhaltenen Hydroxymonocarbonsäuren mit der Epoxidgruppe eines handelsüblichen flüssigen Harzes liefert einen Hydroxyester ohne oder mit geringer Veresterung der Carboxylgruppe mit den sich im gleichen Molekül befindenden Hydroxylgruppen, wie nachstehend dargestellt: 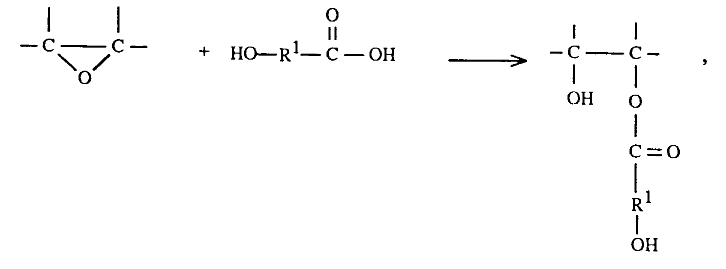
wobei R¹ einen aliphatischen, cycloaliphatischen oder aromatischen Rest, der sich von einem Monohydroxycarbonsäure- oder Polyolanhydrid-Addukt ableitet, bedeutet, und das Addukt durch Reaktion eines Polyols und eines Anhydrids gebildet wird.

Ein Gegenstand der vorliegenden Erfindung sind neue Vernetzungsmittel für verschiedene Systeme.

Ein weiterer Gegenstand vorliegender Erfindung sind Zusammensetzungen für wetterbeständige Beschichtungen und Giessharzteile.

Die vorliegende Erfindung betrifft demnach Polyglycidylester der folgenden Formel (I) 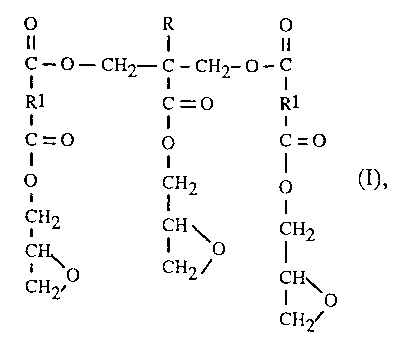
worin R -H oder C₁-C₅-Alkyl ist, und R¹ einen o-Phenylen- oder einen gesättigten oder ungesättigten 1,2-Cyclohexylen-Rest oder einen 2,3-Bicyclo[2.2.1]-hept-5-enylenrest der Formel (II) 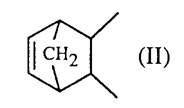
bedeutet, oder R¹ eine unsubstituierte oder durch ein oder mehrere Chlor- oder Bromatome substituierte gesättigte oder ungesättigte aliphatische Gruppe mit 2 bis 20 C-Atomen bedeutet, wobei der o-Phenylenrest unsubstituiert oder durch eine oder zwei C₁-C₄-Alkylgruppen substituiert sein kann, und der 1,2-Cyclohexylenrest und der Bicyclorest der Formel II unsubstituiert oder durch eine oder zwei C₁-C₄-Alkylgruppen und/oder durch ein bis sechs Chlor- oder Bromatome substituiert sein können.

Bevorzugte o-Phenylenreste sind unsubstituiertes oder durch Methyl substituiertes o-Phenylen.

Bevorzugte 1,2-Cyclohexylenreste weisen die folgenden Strukturen 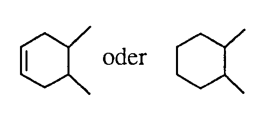
auf, welche unsubstituiert oder durch Methyl, insbesondere in 3,4,5 und 6-Stellung, substituiert sind.

Bevorzugte Reste der Formel II haben die Formeln 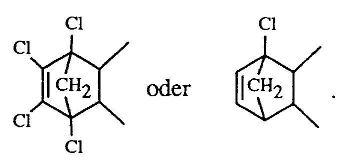
Bevorzugte aliphatische Reste als R¹ weisen von 2 bis 14 C-Atomen auf und sind z.B. -CH₂CH₂-, -CH₂(CH₂)₂CH₂-, CH₂(CH₂)₁₂CH₂- und -CH≡CH-.

R ist bevorzugt -H, -CH₃ oder -C₂H₅, insbesondere aber -CH₃.

Die erfindungsgemässen Polyglycidylester können durch Glycidylierung der Tricarbonsäureaddukte, die aus zwei Mol eines Dicarbonsäureanhydrids und einem Mol 2,2-Dimethylolalkansäure gewonnen werden. Geeignete Tricarbonsäureaddukte, die mit Epichlorhydrin glycidyliert werden können, sind Addukte aus 2,2-Dimethylolalkansäuren und Tetrahydrophthalsäure-, Hexahydrophthalsäure-, Bernsteinsäure-, Maleinsäure-, Dodecenylbernsteinsäure-, Hexachlorendomethylentetrahydrophthalsäure- oder Tetrachlorphthalsäure-anhydrid.

Epoxidierung der so erhaltenen trifunktionellen Carbonsäureaddukte kann unter Verwendung eines Ueberschusses an Epichlorhydrin in Gegenwart eines Phasentransfermittels, z.B. Tetramethylammoniumchlorid, und anschliessende Dehydrohalogenierung mit Natriumhydroxid unter azeotroper Entfernung des Wassers aus dem Reaktionsgemisch zur Reduzierung auf das Minimum einer möglichen Verseifung der Estergruppen durch das alkalische Mittel durchgeführt werden.

Die erfindungsgemäss verwendeten 2,2-Dimethylolalkansäuren weisen 4 bis 9 C-Atome auf und haben die folgende Formel (III): 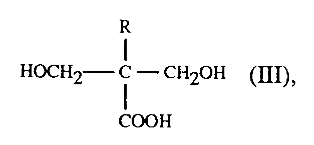
worin R -H oder C₁-C₅-Alkyl bedeutet.

Beispiele hierfür sind 2,2-Dimethylolbuttersäure, 2,2-Dimethylolvaleriansäure und 2,2-Dimethylolcapronsäure, und insbesondere 2,2-Dimethylolpropionsäure (2,2-Bis-hydroxymethylpropionsäure, nachstehend als DMPA abgekürzt).

Ein besonders bevorzugtes DMPA-Addukt mit Hexahydrophthalsäureanhydrid weist die folgende Formel (IV) auf: 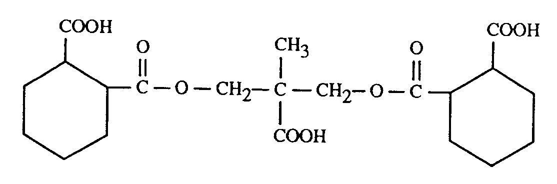
Dieses Addukt ist ein weisses Pulver mit einer Säurezahl von 376 mg KOH/g, und der Glycidylester davon (nachstehende Formel) ist eine viskose klare Flüssigkeit mit einem Epoxidgehalt von 0,35 bis 0,45 Aeq./100 g: 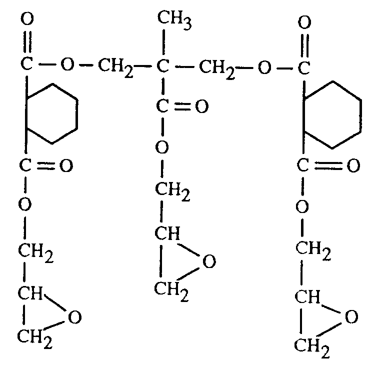
Ein weiterer Gegenstand der vorliegenden Erfindung betrifft eine hitzehärtbare Beschichtungs- und Giessharzzusammensetzung enthaltend
a) einen Polyglycidylester der obigen Formel I und
b) eine effektive Menge eines Vernetzungsmittels aus der Reihe Dicyandiamid, der hitzehärtbaren Polyester enthaltend funktionelle Carboxylgruppe(n), der Polyhydroxyether mit phenolischen Endgruppen, der Amine und der Anhydride.

Wird Dicyandiamid verwendet, so beträgt die effektive Menge des Vernetzungsmittels 2 bis 15 Gewichtsteile für 85 bis 98 Gewichtsteile Polyglycidylester a).

Ist das Vernetzungsmittel ein Polyester mit funktionellen Carboxylgruppen, so beträgt die effektive Menge des Vernetzungsmittels 70 bis 90 Gewichtsteile für 10 bis 30 Gewichtsteile des Polyglycidylesters a).

Beim Einsatz eines Polyhydroxyethers mit phenolischen Endgruppen als Vernetzungsmittel beträgt dessen effektive Menge 10 bis 30 Gewichtsprozente für 70 bis 90 Gewichtsteile des Polyglycidylesters a).

Ist das Vernetzungsmittel ein aliphatisches oder aromatisches Amin, wie z.B. Diaminodiphenylmethan oder 4,4'-Diaminodiphenylsulfon, so werden als effektive Mengen 10 bis 20 Gewichtsteile Vernetzungsmittel für 80 bis 90 Gewichtsteile Polyglycidylester a).

Beim Einsatz eines Anhydrids, wie z.B. Hexahydrophthalsäureanhydrid, so beträgt die effektive Menge des Vernetzungsmittels 35 bis 45 Gewichtsteile für 55 bis 65 Gewichtsteile Polyglycidylester a).

Die erfindungsgemässen Beschichtungszusammensetzungen können auch Lösungsmittel, Pigmente, Füllmittel, das Fliessverhalten regulierende Mittel sowie andere herkömmliche Zusätze für in Beschichtungszusammensetzungen eingesetzte Epoxidharze enthalten.

Die folgenden Beispiele illustrieren den vorliegenden Erfindungsgegenstand.

### Beispiel 1: Herstellung des Adduktes aus DMPA und Hexahydrophthalsäureanhydrid.

Ein 5,0 Liter Kolben mit Rührer, Rückflusskühler, Stickstoffzufuhr und Thermometer wird mit 1850 g Hexahydrophthalsäureanhydrid unter Stickstoff beladen und auf 80°C erwärmt. Dann werden 800 g Methylisobutylketon (MIBK) und anschliessend 805 g Dimethylolpropionsäure (DMPA) und 800 g MIBK zugegeben. Das Reaktionsgemisch wird auf 100°C erwärmt und bei dieser Temperatur während 5,5 Stunden gehalten.

MIBK wird dann bei 60°C unter einem Druck von 6666,1 Pa (50 mm Hg) und anschliessend bei 90°C unter weniger als 1333,22 Pa (10 mm Hg) abdestilliert.

Der Rückstand wird flüssig (als Schmelze) ausgeladen und erkalten lassen. Das erhaltene Produkt ist ein weisses Pulver mit einem Schmelzpunkt von 81°C und einer Säurezahl von 376 mg KOH/g.

### Beispiel 2: Glycidylierung des gemäss Beispiel 1 erhaltenen Adduktes

Ein 5 Liter Kolben mit Rührer, einer azeotropischen Abscheidungseinrichtung für Epichlorhydrin/Wasser und einem Thermometer-Thermoregulator wird mit 1100 g des gemäss Beispiel 1 erhaltenen Adduktes, 5 g Irganox^{®} 1010 (Antioxidanz der Ciba-Geigy Corp.) und 49,3 g einer 50%igen wässrigen Tetramethylammoniumchloridlösung (TMAC) gefüllt. Danach werden 250 g Epichlorhydrin bei 60°C zugetropft, und das Gemisch wird während 30 Minuten weitergerührt, wobei die Temperatur durch Kühlung, falls nötig, auf 60-65°C gehalten wird.

Dann werden 49,3 g einer 50%igen TMAC-Lösung zugegeben, und Teilvakuum von ca. 6666,1 Pa (ca. 50 mm Hg) wird zur Einstellung des Rückflusses (ca 65°C) angelegt.

Anschliessend werden 540 g einer 50%igen NaOH-Lösung innerhalb von 4 bis 5 Stunden zugegeben, wobei gleichzeitig Wasser durch azeotropische Destillation entfernt wird. Nach der Zugabe der NaOH-Lösung wird das restliche Wasser während 7 Stunden entfernt, das Vakuum wird ausgeschaltet und der Kolben wird auf Raumtemperatur abgekühlt. Der Rückstand wird vom Salz durch Filtration befreit, das Filtrat wird zweimal mit 750 ml einer 10%igen warmen wässrigen Natriumzitratlösung während 5 Minuten extrahiert, das erhaltene Gemisch wird 10 Minuten stehen gelassen, die obere wässrige Schicht wird entfernt und die organische Schicht wird filtriert.

Die Aufarbeitung wird wie folgt durchgeführt:
1. Die organische Schicht mehrerer Ansätze wird gesammelt und in einem 12,0 Liter Drei-Halsrundkolben mit Vakuumdestillationseinrichtung abgefüllt.
2. 262 g einer 50%igen wässrigen NaOH-Lösung und 105 g einer 50%igen wässrigen TMAC-Lösung werden zugegeben, und die erhaltene Mischung wird auf 70°C erwärmt und während 30 Min. bei dieser Temperatur gehalten.
3. 2000 ml deionisiertes Wasser werden zugegeben, und das Gemisch wird auf 70°C erwärmt, so dass sich der gebildete Niederschlag wieder löst.
4. Die organische (obere) Schicht wird abdekantiert und mit 2000 ml deionisiertem Wasser und 20 g trockenem Eis gewaschen. Das gesamte Gemisch wird auf 70°C zur vollständigen Lösung eines allfälligen Niederschlages erwärmt.
5. Der pH-Wert der wässrigen Phase (obere Schicht) des zweiten Waschgangs muss unter 7 sein.
6. Die organische (untere) Schicht wird gesammelt, mit 100 g Supergel Hyflo, dann mit 3000 ml MIBK behandelt, und anschliessend vom Supergel abfiltriert.
7. MIBK wird dann bei 60°C unter Druck (10 mm Hg) abdestilliert.

Ausbeute: 68 % der Theorie [2-Methyl-2-ketoglycidyloxy-1,3-ketoxy-bis(β-ketoglycidyloxy-α-cyclohexyl)propan].

Das erhaltene Produkt weist folgende Eigenschaften auf:
Prozentige Epoxidierung: 77;
Epoxidgehalt: 0,414 Aeq/100 g;
Säurezahl: <1;
Viskosität bei 25°C: 16800 cP;
Chlorgehalt (total): 1,50 %;
Hydrolysierbares Chlor: 1,00 %.

## Patentansprüche (Patentansprüche für folgende(n) Vertragsstaat(en): DE, GB, FR, IT, CH, LI)

1. Verbindungen der Formel (I) 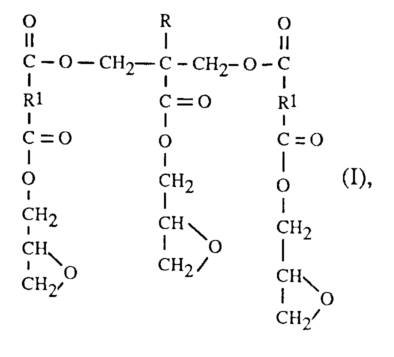 worin R -H oder C₁-C₅-Alkyl ist, und R¹ einen o-Phenylen- oder einen gesättigten oder ungesättigten 1,2-Cyclohexylen-Rest oder einen 2,3-Bicyclo[2.2.1]-hept-5-enylenrest der Formel II 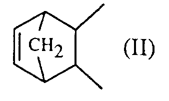 bedeutet, oder R¹ eine unsubstituierte oder durch ein oder mehrere Chlor- oder Bromatome substituierte gesättigte oder ungesättigte aliphatische Gruppe mit 2 bis 20 C-Atomen bedeutet, wobei der o-Phenylenrest unsubstituiert oder durch eine oder zwei C₁-C₄-Alkylgruppen substituiert sein kann, und der 1,2-Cyclohexylrest und der Bicyclorest der Formel II unsubstituiert oder durch eine oder zwei C₁-C₄-Alkylgruppen und/oder durch ein bis sechs Chlor- oder Bromatome substituiert sein können.

2. Verbindungen gemäss Anspruch 1, worin R Methyl ist.

3. Verbindungen gemäss Anspruch 1, worin R¹ als aliphatische Gruppe 2 bis 14 C-Atome aufweist.

4. Verbindungen gemäss Anspruch 1, worin die o-Phenylengruppe unsubstituiert oder durch Methyl substituiert ist.

5. Verbindungen gemäss Anspruch 1, worin der 1,2-Cyclohexylenrest eine Gruppe der Formeln 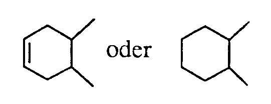 darstellt, welche unsubstituiert oder durch Methyl substituiert sind.

6. Verbindungen gemäss Anspruch 1, worin der Bicyclorest der Formel II eine Gruppe der Formeln 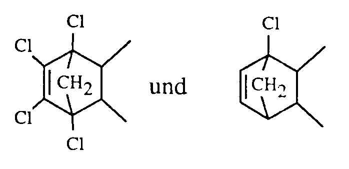 darstellt.

7. Verbindungen gemäss Anspruch 1, worin R¹ als aliphatische Gruppe eine Gruppe der Formeln -CH₂CH₂-, -CH₂(CH₂)₂CH₂-, -CH₂(CH₂)₁₂CH₂- oder -CH≡CH- ist.

8. Verbindungen der Formel (V) gemäss Anspruch 1:

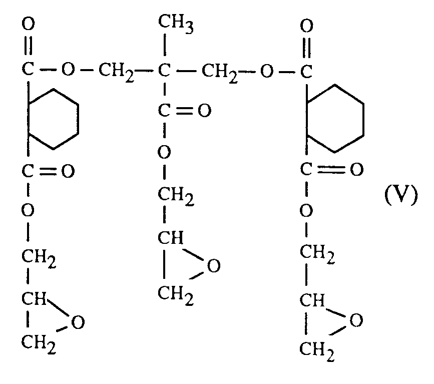

9. Hitzehärtbare Beschichtungszusammensetzungen enthaltend
a) einen Polyglycidylester der Formel (I) gemäss Anspruch 1 und
b) eine effektive Menge eines Vernetzungsmittels aus der Reihe Dicyandiamid, der hitzehärtbaren Polyester enthaltend polyfunktionelle Carbonsäure(n), der Polyhydroxyether mit phenolischen Endgruppen, der Amine und der Anhydride.

10. Zusammensetzungen gemäss Anspruch 9, worin der Polyglycidylester die Formel (V) 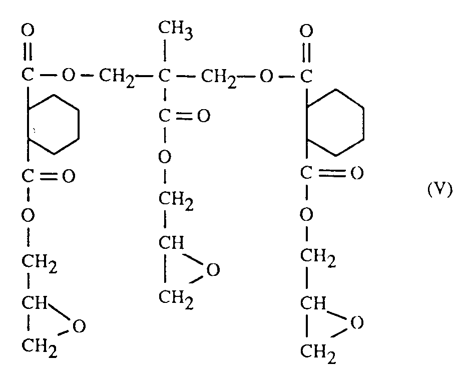 aufweist.

11. Zusammensetzungen gemäss Anspruch 9, worin das Vernetzungsmittel Dicyandiamid ist.

12. Zusammensetzungen gemäss Anspruch 11, worin die effektive Menge des Vernetzungsmittels 2 bis 15 Gewichtsteile für 85 bis 98 Gewichtsteile Polyglycidylester beträgt.

13. Zusammensetzungen gemäss Anspruch 9, worin das Vernetzungsmittel ein hitzehärtbarer Polyester mit funktionellen Carboxylgruppen ist.

14. Zusammensetzungen gemäss Anspruch 13, worin die effektive Menge des Vernetzungsmittels 70 bis 90 Gewichtsteile für 10 bis 30 Gewichtsteile Polyglycidylester beträgt.

15. Zusammensetzungen gemäss Anspruch 9, worin das Vernetzungsmittel ein Polyhydroxyether mit phenolischen Endgruppen ist.

16. Zusammensetzungen gemäss Anspruch 15, worin die effektive Menge des Polyhydroxyethers 10 bis 30 Gewichtsteile für 70 bis 90 Gewichtsteile des Polyglycidylesters beträgt.

17. Zusammensetzungen gemäss Anspruch 9, worin das Vernetzungsmittel ein aromatisches oder aliphatisches Amin ist.

18. Zusammensetzungen gemäss Anspruch 17, worin die effektive Menge des Amins 10 bis 20 Gewichtsteile für 80 bis 90 Gewichtsteile Polyglycidylester beträgt.

19. Zusammensetzungen gemäss Anspruch 9, worin in der Formel 1 R Methyl bedeutet.

20. Zusammensetzungen gemäss Anspruch 9, worin in der Formel I R¹ 1,2-Cyclohexen bedeutet.

## Patentansprüche (Patentansprüche für folgende(n) Vertragsstaat(en): ES)

1. Hitzehärtbare Beschichtungszusammensetzungen enthaltend
a) einen Polyglycidylester der nachstehenden Formel (I) und
b) eine effektive Menge eines Vernetzungsmittels aus der Reihe Dicyandiamid, der hitzehärtbaren Polyester enthaltend polyfunktionelle Carbonsäure(n), der Polyhydroxyether mit phenolischen Endgruppen, der Amine und der Anhydride 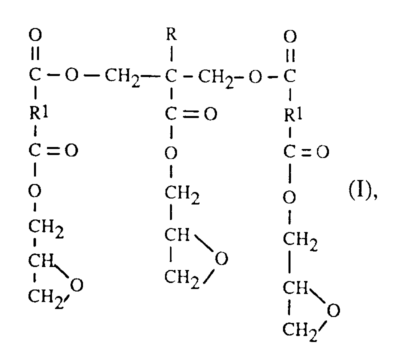 worin R -H oder C₁-C₅-Alkyl ist, und R¹ einen o-Phenylen- oder einen gesättigten oder ungesättigten 1,2-Cyclohexylen-Rest oder einen 2,3-Bicyclo[2.2.1]-hept-5-enylenrest der Formel II 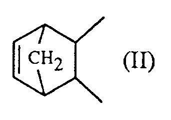 bedeutet, oder R¹ eine unsubstituierte oder durch ein oder mehrere Chlor- oder Bromatome substituierte gesättigte oder ungesättigte aliphatische Gruppe mit 2 bis 20 C-Atomen bedeutet, wobei der o-Phenylenrest unsubstituiert oder durch eine oder zwei C₁-C₄-Alkylgruppen substituiert sein kann, und der 1,2-Cyclohexylrest oder der Bycyclorest der Formel II unsubstituiert oder durch eine oder zwei C₁-C₄-Alkylgruppen und/oder durch ein bis sechs Chlor- oder Bromatome substituiert sein können.

2. Zusammensetzungen gemäss Anspruch 1, worin der Polyglycidylester die Formel (V) 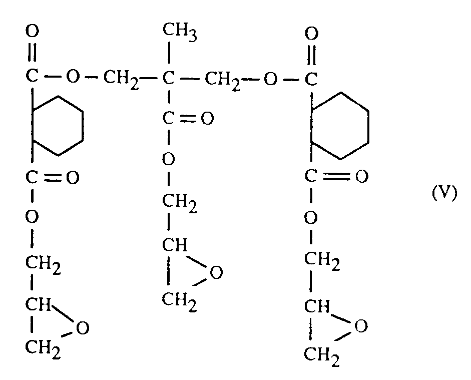 aufweist.

3. Zusammensetzungen gemäss Anspruch 1, worin das Vernetzungsmittel Dicyandiamid ist.

4. Zusammensetzungen gemäss Anspruch 1, worin die effektive Menge des Vernetzungsmittels 2 bis 15 Gewichtsteile für 85 bis 98 Gewichtsteile Polyglycidylester beträgt.

5. Zusammensetzungen gemäss Anspruch 1, worin das Vernetzungsmittel ein hitzehärtbarer Polyester mit funktionellen Carboxylgruppen ist.

6. Zusammensetzungen gemäss Anspruch 5, worin die effektive Menge des Vernetzungsmittels 70 bis 90 Gewichtsteile für 10 bis 30 Gewichtsteile Polyglycidylester beträgt.

7. Zusammensetzungen gemäss Anspruch 1, worin das Vernetzungsmittel ein Polyhydroxyether mit phenolischen Endgruppen ist.

8. Zusammensetzungen gemäss Anspruch 7, worin die effektive Menge des Polyhydroxyethers 10 bis 30 Gewichtsteile für 70 bis 90 Gewichtsteile des Polyglycidylesters beträgt.

9. Zusammensetzungen gemäss Anspruch 1, worin das Vernetzungsmittel ein aromatisches oder aliphatisches Amin ist.

10. Zusammensetzungen gemäss Anspruch 9, worin die effektive Menge des Amins 10 bis 20 Gewichtsteile für 80 bis 90 Gewichtsteile Polyglycidylester beträgt.

11. Zusammensetzungen gemäss Anspruch 1, worin in der Formel I R Methyl bedeutet.

12. Zusammensetzungen gemäss Anspruch 1, worin in der Formel I R¹ 1,2-Cyclohexen bedeutet.

## Claims (Claims for the following Contracting State(s): DE, GB, FR, IT, CH, LI)

1. A compound of the formula (I) 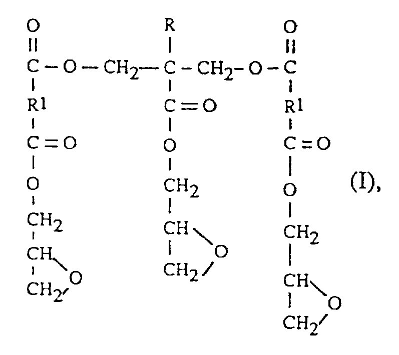 wherein R is hydrogen or C₁-C₅alkyl and
R¹ is o-phenylene, a saturated or unsaturated 1,2-cyclohexylene radical or a 2,3-bicyclo[2.2.1]hept-5-enylene radical of the formula (II) 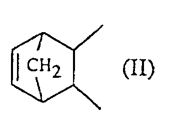 or R¹ is a saturated or unsaturated aliphatic group of 2 to 20 carbon atoms which is unsubstituted or mono- or polysubstituted by chlorine or bromine atoms, the o-phenylene radical being able to be unsubstituted or substituted by one or two C₁-C₄alkyl groups, and the 1,2-cyclohexyl radical and the bicyclo radical of the formula II being able to be unsubstituted or substituted by one or two C₁-C₄alkyl groups and/or by one to six chlorine or bromine atoms.

2. A compound according to claim 1 wherein R is methyl.

3. A compound according to claim 1 wherein R¹ as aliphatic group has 2 to 14 carbon atoms.

4. A compound according to claim 1 wherein the o-phenylene group is unsubstituted or methyl-substituted.

5. A compound according to claim 1 wherein the 1,2-cyclohexylene radical is a group of the formula 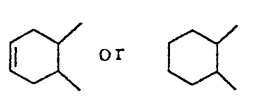 which are unsubstituted or methyl-substituted.

6. A compound according to claim 1 wherein the bicyclo radical of the formula II is a group of the formulae

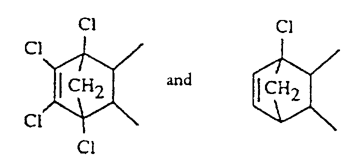

7. A compound according to claim 1 wherein R¹ as aliphatic group is a group of the formula -CH₂CH₂-, -CH₂(CH₂)₂CH₂-, -CH₂(CH₂)₁₂CH₂- or -CH=CH-.

8. A compound according to claim 1 of the formula (V):

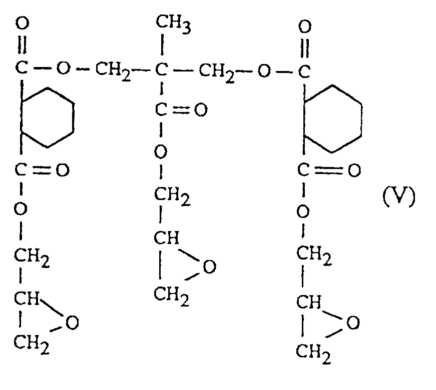

9. A thermosetting coating composition comprising
a) a polyglycidyl ester of the formula (I) according to claim 1 and
b) an effective amount of a crosslinking agent selected from the series consisting of dicyandiamide, thermosetting polyesters containing polyfunctional carboxylic acid(s), polyhydroxyethers with phenolic end groups, amines and anhydrides.

10. A composition according to claim 9 wherein the polyglycidyl ester is of the formula (V)

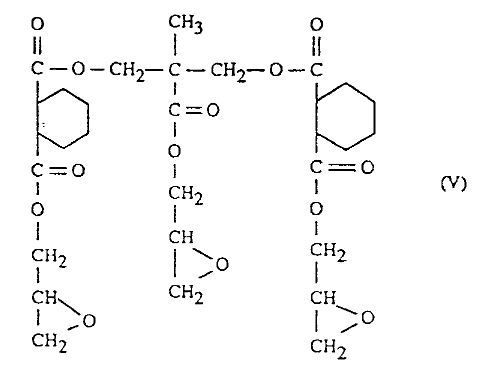

11. A composition according to claim 9 wherein the crosslinking agent is dicyandiamide.

12. A composition according to claim 11 wherein for 85 to 98 parts by weight of polyglycidyl ester the effective amount of crosslinking agent is 2 to 15 parts by weight.

13. A composition according to claim 9 wherein the crosslinking agent is a thermosetting polyester which has functional carboxyl groups.

14. A composition according to claim 13 wherein for 10 to 30 parts by weight of polyglycidyl ester the effective amount of crosslinking agent is 70 to 90 parts by weight.

15. A composition according to claim 9 wherein the crosslinking agent is a polyhydroxyether with phenolic end groups.

16. A composition according to claim 15 wherein for 70 to 90 parts by weight of polyglycidyl ester the effective amount of polyhydroxyether is 10 to 30 parts by weight.

17. A composition according to claim 9 wherein the crosslinking agent is an aromatic or aliphatic amine.

18. A composition according to claim 17 wherein for 80 to 90 parts by weight of polyglycidyl ester the effective amount of amine is 10 to 20 parts by weight.

19. A composition according to claim 9 wherein R is methyl in the formula I.

20. A composition according to claim 9 wherein R¹ is 1,2-cyclohexylene in the formula I.

## Claims (Claims for the following Contracting State(s): ES)

1. A thermosetting coating composition comprising
a) a polyglycidyl ester of the formula (I) below and
b) an effective amount of a crosslinking agent selected from the series consisting of dicyandiamide, thermosetting polyesters containing polyfunctional carboxylic acid(s), polyhydroxyethers with phenolic end groups, amines and anhydrides; 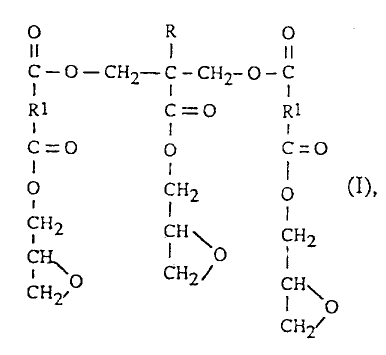 wherein R is hydrogen or C₁-C₅alkyl and
R¹ is o-phenylene, a saturated or unsaturated 1,2-cyclohexylene radical or a 2,3-bicyclo[2.2.1]hept-5-enylene radical of the formula (II) 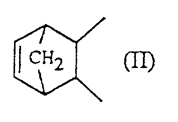 or R¹ is a saturated or unsaturated aliphatic group of 2 to 20 carbon atoms which is unsubstituted or mono- or polysubstituted by chlorine or bromine atoms, the o-phenylene radical being able to be unsubstituted or substituted by one or two C₁-C₄alkyl groups, and the 1,2-cyclohexyl radical and the bicyclo radical of the formula II being able to be unsubstituted or substituted by one or two C₁-C₄alkyl groups and/or by one to six chlorine or bromine atoms.

2. A composition according to claim 1 wherein the polyglycidyl ester is of the formula (V)

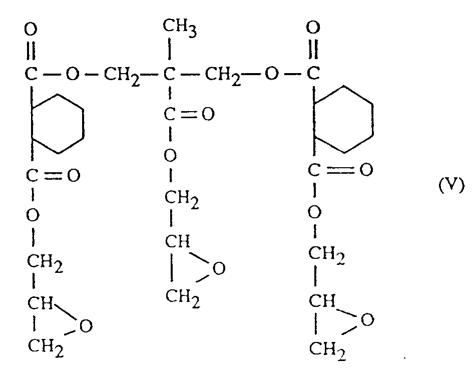

3. A composition according to claim 1 wherein the crosslinking agent is dicyandiamide.

4. A composition according to claim 1 wherein for 85 to 98 parts by weight of polyglycidyl ester the effective amount of crosslinking agent is 2 to 15 parts by weight.

5. A composition according to claim 1 wherein the crosslinking agent is a thermosetting polyester which has functional carboxyl groups.

6. A composition according to claim 5 wherein for 10 to 30 parts by weight of polyglycidyl ester the effective amount of crosslinking agent is 70 to 90 parts by weight.

7. A composition according to claim 1 wherein the crosslinking agent is a polyhydroxyether with phenolic end groups.

8. A composition according to claim 7 wherein for 70 to 90 parts by weight of polyglycidyl ester the effective amount of polyhydroxyether is 10 to 30 parts by weight.

9. A composition according to claim 1 wherein the crosslinking agent is an aromatic or aliphatic amine.

10. A composition according to claim 9 wherein for 80 to 90 parts by weight of polyglycidyl ester the effective amount of amine is 10 to 20 parts by weight.

11. A composition according to claim 1 wherein R is methyl in the formula I.

12. A composition according to claim 1 wherein R¹ is 1,2-cyclohexylene in the formula I.

## Revendications (Revendications pour l'(les) Etat(s) contractant(s) suivant(s): DE, GB, FR, IT, CH, LI)

1. Composés de formule I 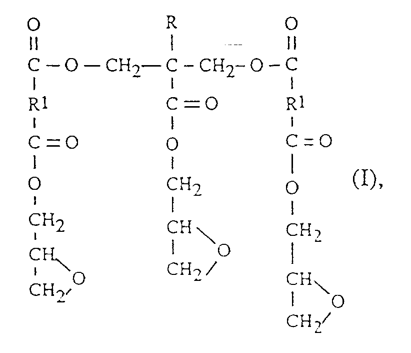 dans laquelle R représente l'hydrogène ou un alkyle en C₁-C₅ et R¹ un radical o-phénylène, un radical 1,2-cyclohexylène saturé ou insaturé ou un radical 2,3-bicyclo-[2.2.1]-hept-5-énylène de formule II : 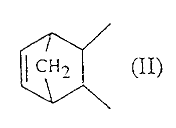 ou R¹ représente un groupe aliphatique saturé ou insaturé pouvant avoir de 2 à 20 atomes de carbone, non substitué ou substitué par un ou plusieurs atomes de chlore ou de brome, le radical o-phénylène pouvant être non substitué ou substitué par un ou deux groupes alkyle en C₁-C₄, et le radical 1,2-cyclohexyl(èn)e ainsi que le radical bicyclique de formule II pouvant être non substitués ou substitués par un ou deux groupes alkyle en C₁-C₄ et/ou par 1 à 6 atomes de chlore ou de brome.

2. Composés selon la revendication 1, dans lesquels R est le groupe méthyle.

3. Composés selon la revendication 1, dans lesquels R¹, en tant que groupe aliphatique, a de 2 à 14 atomes de carbone.

4. Composés selon la revendication 1, dans lesquels le groupe o-phénylène est non substitué ou substitué par le méthyle.

5. Composés selon la revendication 1, dans lesquels le radical 1,2-cyclohexène est un groupe de formule 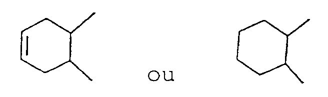 qui est non substitué ou substitué par le méthyle.

6. Composés selon la revendication 1, dans lesquels le radical bicyclique de formule II est un groupe de formule(s)

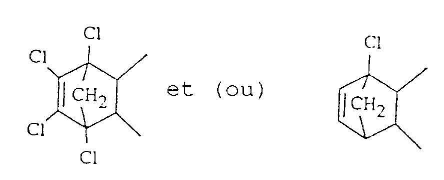

7. Composés selon la revendication 1, dans lesquels R¹, en tant que groupe aliphatique, est un groupe -CH₂CH₂-, -CH₂(CH₂)₂CH₂-, CH₂(CH₂)₁₂CH₂- ou -CH≡CH-.

8. Composé de formule V selon la revendication 1 :

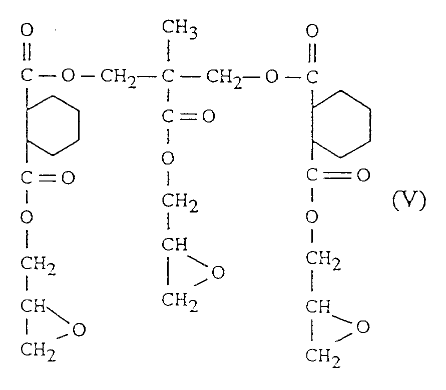

9. Compositions de revêtement thermodurcissables qui comprennent :
a) un ester polyglycidylique de formule I selon la revendication 1 et
b) une quantité efficace d'un agent réticulant de la série du dicyandiamide, des polyesters thermodurcissables avec un ou plusieurs groupe(s) carboxylique (s) polyfonctionnel(s) des polyhydroxyéthers à groupes terminaux phénoliques, des amines et des anhydrides.

10. Compositions selon la revendication 9 dans lesquelles l'ester polyglycidylique a la formule V

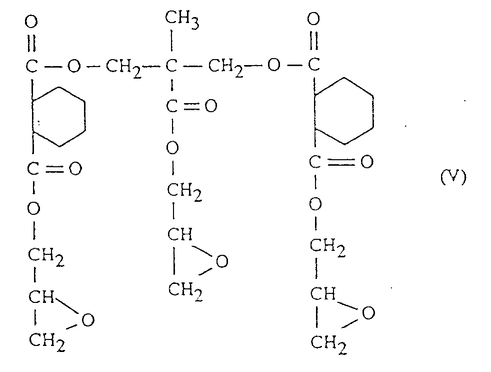

11. Compositions selon la revendication 9, dans lesquelles l'agent réticulant est le dicyandiamide.

12. Compositions selon la revendication 11, dans lesquelles la quantité efficace de l'agent réticulant est de 2 à 15 parties en poids pour 85 à 98 parties en poids d'ester polyglycidylique.

13. Compositions selon la revendication 9, dans lesquelles l'agent réticulant est un polyester thermodurcissable à groupes carboxyliques fonctionnels.

14. Compositions selon la revendication 13, dans lesquelles la quantité efficace de l'agent réticulant est de 70 à 90 parties en poids pour 10 à 30 parties en poids d'ester polyglycidylique.

15. Compositions selon la revendication 9, dans lesquelles l'agent réticulant est un polyhydroxyéther à groupes terminaux phénoliques.

16. Compositions selon la revendication 15, dans lesquelles la quantité efficace du polyhydroxyéther est de 10 à 30 parties en poids pour 70 à 90 parties en poids de l'ester polyglycidylique.

17. Compositions selon la revendication 9, dans lesquelles l'agent réticulant est une amine aromatique ou aliphatique.

18. Compositions selon la revendication 17, dans lesquelles la quantité efficace de l'amine est de 10 à 20 parties en poids pour 80 à 90 parties en poids d'ester polyglycidylique.

19. Compositions selon la revendication 9, dans lesquelles dans la formule I, R est le groupe méthyle.

20. Compositions selon la revendication 9, dans lesquelles dans la formule I, R¹ est la groupe 1,2-cyclohexène.

## Revendications (Revendications pour l'(les) Etat(s) contractant(s) suivant(s): ES)

1. Compositions de revêtement thermo-durcissables qui comprennent :
a) un ester polyglycidylique de formule I ci-après, et
b) une quantité efficace d'un agent réticulant de la série du dicyandiamide, des polyesters thermo-durcissables avec un ou plusieurs groupe(s) carboxylique(s) polyfonctionnel(s), des polyhydroxyéthers à groupes terminaux phénoliques, des amines et des anhydrides : 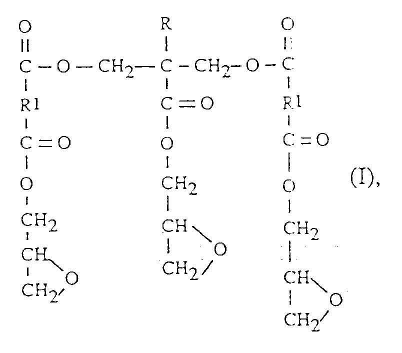 dans laquelle R représente l'hydrogène ou un alkyle en C₁-C₅ et R¹ un radical o-phénylène, un radical 1,2-cyclohexylène saturé ou insaturé ou un radical 2,3-bicyclo-[2.2.1]-hept-5-énylène de formule II : 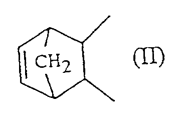 ou R¹ représente un groupe aliphatique saturé ou insaturé pouvant avoir de 2 à 20 atomes de carbone, non substitué ou substitué par un ou plusieurs atomes de chlore ou de brome, le radical o-phénylène pouvant être non substitué ou substitué par un ou deux groupes alkyle en C₁-C₄, et le radical 1,2-cyclohexyl(èn)e ainsi que le radical bicyclique de formule II pouvant être non substitués ou substitués par un ou deux groupes alkyle en C₁-C₄ et/ou par 1 à 6 atomes de chlore ou de brome.

2. Compositions selon la revendication 1, dans lesquelles l'ester polyglycidylique a la formule V

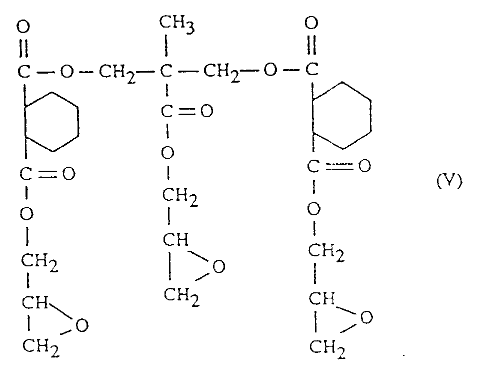

3. Compositions selon la revendication 1, dans lesquelles l'agent réticulant est le dicyandiamide.

4. Compositions selon la revendication 1, dans lesquelles la quantité efficace de l'agent réticulant est de 2 à 15 parties en poids pour 85 à 98 parties en poids d'ester polyglycidylique.

5. Compositions selon la revendication 1, dans lesquelles l'agent réticulant est un polyester thermodurcissable à groupes carboxyliques fonctionnels.

6. Compositions selon la revendication 5, dans lesquelles la quantité efficace de l'agent réticulant est de 70 à 90 parties en poids pour 10 à 30 parties en poids d'ester polyglycidylique.

7. Compositions selon la revendication 1, dans lesquelles l'agent réticulant est un polyhydroxyéther à groupes terminaux phénoliques.

8. Compositions selon la revendication 7, dans lesquelles la quantité efficace du polyhydroxyéther est de 10 à 30 parties en poids pour 70 à 90 parties en poids de l'ester polyglycidylique.

9. Compositions selon la revendication 1, dans lesquelles l'agent réticulant est une amine aromatique ou aliphatique.

10. Compositions selon la revendication 9, dans lesquelles la quantité efficace de l'amine est de 10 à 20 parties en poids pour 80 à 90 parties en poids d'ester polyglycidylique.

11. Compositions selon la revendication 1, dans lesquelles dans la formule I, R est le groupe méthyle.

12. Compositions selon la revendication 1, dans lesquelles dans la formule I, R¹ est le groupe 1,2-cyclohexène.
